# EUROPEAN PATENT APPLICATION

(11) **EP 1 452 162 A1**
(43) Date of publication of application: **01.09.2004**
(21) Application number: 02803101.1
(22) Date of filing: 11.11.2002
(51) Int. Cl.: A61K 7/00, A61K 7/48, A61K 9/06, A61K 47/14, A61K 47/24

(54) **GEL-FORM COMPOSITION**

(30) Priority: 09.11.2001 JP 2001345351
(71) Applicant: THE NISSHIN OIL MILLS, LTD., Tokyo, 104-8285 (JP)
(72) Inventor: TOYODA, Ryoichi c/o The Nisshin OilliO, Ltd., Yokohama-shi, Kanagawa 235- (JP); OYAMA, Keiichi c/o The Nisshin OilliO, Ltd., Isogo-ku Yokohama-shi, Kanagawa 235-8 (JP)
(74) Representative: Casalonga, Axel
(86) International application number: PCT/JP2002/011717
(87) International publication number: WO 2003/041665

(57) **Abstract**

A gel-form composition comprising the following ingredients (A), (B) and (C) as indispensable ingredients, the compounding amount of lecithin in the gel-form composition being 1 % by mass or more:
(A) a polyglycerol fatty acid ester having an HLB value of 8 or more, each fatty acid residue thereof having 14 to 22 carbon atoms,
(B) lecithin, and
(C) water.

The gel-form composition of the invention can be used in the fields of cosmetics, quasi-medicaments, medicaments, household utensils, etc. and therein, polyglycerol fatty acid esters and lecithin are used as bases. The gel-form composition of the invention has excellent abilities to prevent secondary adhesion after external use and, moreover, retains stable quality regardless of the purity of phosphatidylcholine in the lecithin.

## Description

### TECHNICAL FIELD

This invention relates to a gel-form composition which is used in the fields of cosmetics, quasi- medicaments, medicaments, household utensils, etc. and wherein polyglycerol fatty acid esters and lecithin are used as bases, and in detail, relates to such a gel-form composition that secondary adhesion after external use is prevented and its stable quality is retained regardless of the purity of phosphatidylcholine in the lecithin. Said secondary adhesion after external use means that when a part on which an external preparation or the like was applied is contacted with another thing, part of the external preparation or the like of the coated part moves and adheres onto the thing.

### BACKGROUND ART

Recently, many cosmetics aiming at moisturizing are put on the market, various products in which various moisturizing ingredients are compounded are provided from various companies. As moisturizing ingredients, there can be mentioned polyols such as glycerol and polyethylene glycol; NMF (natural moisturizing factor) ingredients such as pyrrolidonecarboxylic acid and urea; oily ingredients such as vegetable oils, ester oils, mineral oils and silicone oils; surfactants such as lecithin and polyglycerol fatty acid esters; etc.

Moisturizing effect includes not only supply of moisture onto the skin but also inhibition of transpiration of the moisture on the skin, and oily ingredients are very useful, because of its occlusive properties, as emollients which inhibit transpiration of the moisture. Thus, oily ingredients are widely used in W/O and O/W emulsion cosmetics aiming at moisturizing.

It is known that lecithin forms lamella liquid crystals by interaction with water and retains a large amount of water, and thus shows moisturizing properties equal to or more than those of oily ingredients. Therefore, in a system where lecithin is used, it is possible to provide a preparation which does not contain any oily ingredient or contains only a small amount of oily ingredients and yet shows high moisturizing properties and are thus free of oily touch.

Furthermore, although since lecithin also has surface active properties, it is widely used as a base of moisturizing cosmetics, it is known that the function of lecithin as a surfactant is largely influenced depending on the purity of phosphatidylcholine contained in the lecithin.

Many studies have been made on moisturizing cosmetics using lecithin as a base. It is known that generally by mixing' hydrogenated lecithin, water and a surfactant supplementing the surface active properties of the hydrogenated lecithin, a gel-form composition in which the improvement of the touch of lecithin itself such as stickiness and the touch of thick membrane is intended can be obtained (JP-A-1-261317).

However, on the one hand, studies to improve the ability and sustainability of moisturizing and touch at the time of use progress on gel-form compositions using lecithin as a base, but, on the other hand, there is a growing need for moisturizing cosmetics free of secondary adhesion after external use, in work under an environment which tends to be dry. For example, under such an environment that humans need to wash their hands frequently, as is the case of housewives, there is a method of coating their hands with an external, preparation for preventing their hands from drying, but there are many case where it is undesirable to apply an external preparation to parts which will contact with other things (dishwashing, etc.). In addition, there are also many demands for such moisturizing cosmetics from special professional fields such as medical sites.

The aforementioned oily O/W-type creams, etc. are not suitable for such demands because oily ingredients are compounded.

It can hardly be said that gel-form compositions using lecithin as a base which have so far been developed, including ones in which touch at the time of use was improved, can prevent secondary adhesion. For example, in a system where, in addition to lecithin, an ethylene oxide-type surfactant is compounded, the improvement of touch of thick membrane and stickiness is intended, but no consideration is made on secondary adhesion, and according to the experiments by the present inventors, secondary adhesion rather increases, compared with a system where no surfactant is compounded (comparison between Comparative examples 4 to 6 and Comparative examples 7 to 9 described later). Furthermore, when an ethylene oxide-type surfactant is used, there is a problem that change of the phase state in proportion to temperature rise is large.

Furthermore, another problem of general gel-form compositions using lecithin as a base is that the quality (e.g., viscosity) of the products tends to be influenced by the purity of phosphatidylcholine in the lecithin. Generally available lecithin is a natural product and thus has dispersion in the concentration of phosphatidylcholine, and there is a problem that the viscosity of the resulting products tends to disperse every lot. Namely, the purity of phosphatidylcholine is different from lot to lot, and this gives a large difference in quality.

### DISCLOSURE OF INVENTION

Thus, the object of the invention is to provide a gel-form composition using lecithin as a base which can prevent secondary adhesion after external use and has stable quality even when there is a difference in the purity of phosphatidylcholine contained in lecithin.

The present inventors intensely studied for solving the above problems, and as a result, they found that a gel-form composition using a particular polyglycerol fatty acid ester, lecithin and water as bases can solve the above problems, and completed this invention.

Namely, this invention relates to a gel-form composition comprising the following ingredients (A), (B) and (C) as indispensable ingredients, the content of lecithin in the gel-form composition being 1 % by mass or more:
(A) a polyglycerol fatty acid ester having an HLB value of 8 or more, each fatty acid residue thereof having 14 to 22 carbon atoms,
(B) lecithin, and
(C) water.

### BEST MODE FOR CARRYING OUT THE INVENTION

The polyglycerol fatty acid ester as an indispensable ingredient of the gel-form composition of the invention is a polyglycerol fatty acid ester which has an HLB value of 8 or more and wherein each fatty acid residue has 14 to 22 carbon atoms. Such fatty acid may be a saturated fatty acid, or an unsaturated fatty acid having one or more, preferably 1 to 3, more preferably 1 or 2 carbon-carbon double bonds.

When the HLB value of the polyglycerol fatty acid ester is less than 8, the surface active ability of lecithin cannot sufficiently be supplemented, and the lecithin gets hard to disperse uniformly. When each fatty acid residue is one having 14 to 22 carbon atoms, the gel-form composition shows stable quality over a wide range of temperature, but when each fatty acid residue is one having less than 14 carbon atoms, e.g., a lauroyl group or a decanoyl group; the gel-form composition gets to be poor in stability particularly at 40 °C or more. Therefore, a general gel-form composition can be obtained even if such a polyglycerol fatty acid ester that the .. HLB value or the carbon number of each fatty acid residue is out of the above-mentioned range is used, but it gets to be difficult to prevent secondary adhesion after external use, and the gel-form composition gets liable to suffer influence due to difference in the purity of phosphatidylcholine contained in the lecithin, and stable quality gets hard to obtain.

In the above, quality being stable means that appearances (texture, luster, uniformity, etc.) and physical properties (viscosity, etc.) are maintained in a good constant state. Additionally, the reason why it is needed that the viscosity of the gel-form composition is constant is that if the viscosity is different every lot of the same product, easiness of application (it flows if it is too soft; it gets difficult to take out of the vessel easily, if it is too hard, etc.) and touch of application (goodness of spread, etc.) get different among the. same products, which may cause a claim from customers.

As examples of polyglycerol fatty acid esters usable in the invention, there can be mentioned triglycerol monomyristate, pentaglycerol monomyristate, hexaglycerol monomyristate, decaglycerol monomyristate, pentaglycerol trimyristate, decaglycerol monopalmitate, triglycerol monostearate, tetraglycerol monostearate, pentaglycerol monostearate, hexaglycerol monostearate, octaglycerol monostearate, decaglycerol monostearate, hexaglycerol distearate, decaglycerol distearate, decaglycerol tristearate, triglycerol monooleate, tetraglycerol monooleate, pentaglycerol monooleate, hexaglycerol monooleate, hexaglycerol dioleate, octaglycerol monooleate, decaglycerol monooleate, decaglycerol dioleate, decaglycerol trioleate, decaglycerol monolinolate, triglycerol monoisostearate, tetraglycerol monoisostearate, pentaglycerol monoisostearate, hexaglycerol monoisostearate, decaglycerol monoisostearate, hexaglycerol diisostearate, decaglycerol diisostearate, etc. These can be used alone or in combination of two or more.

Preferably, polyglycerol fatty acid esters used in the invention are such that the average polymerization degree of the constitutive polyglycerols is 3 to 12. When the average polymerization degree is less than 3, the hydrophilicity of the polyglycerol fatty acid esters is lowered, which turns uniform dispersion of lecithin difficulat, and when it is more than 12, such problems are liable to arise that the texture of the gel-form composition gets bad, hardness is hard to give and/or stability at high temperatures is lowered.

Furthermore, it is preferred that ingredient (A) as the polyglycerol fatty acid ester used in the invention contains 1 % by mass or more of the total mass of ingredient (A) of such a polyglycerol'fatty acid ester mixture that the total content of polyglycerol fatty acid esters whose constitutive polyglycerols are pentamers of glycerol and polyglycerol fatty acid esters whose constitutive polyglycerols are hexamers of glycerol is 50 % by mass or more and the total content of polyglycerol fatty acid esters whose constitutive polyglycerols are tetramers and less oligomers of glycerol is less than 20 % by mass. In the above, as to the polyglycerol fatty acid ester mixture, it is further preferred that the total content of pentamers of glycerol-based polyglycerol fatty acid esters and hexamers of glycerol-based polyglycerol fatty acid esters is 70 % by mass or more, it is further preferred that the total content of tetramers and less oligomers of glycerol-based polyglycerol fatty acid esters is less than 10 % by mass, and it is still further preferred that the total content of the former is 70 % by mass or more and at the same time the total content of the latter is less than 10 % by mass. Furthermore, in the above, the content of the polyglycerol fatty acid ester mixture in ingredient (A) is more preferably 10 % by mass or more, still more preferably 50 % by mass or more and most preferably 80 % by mass or more, particularly 100 % by mass.

In the above, as to the polyglycerol fatty acid ester mixture, when the total content of pentamers of glycerol-based polyglycerol fatty acid esters and hexamers of glycerol-based polyglycerol fatty acid esters is less than 50 % by mass, or the total content of tetramers and less oligomers of glycerol-based polyglycerol fatty acid esters is more than 20 % by mass, it tends to get difficult to maintain the hardness of the gel-form composition and the stability of the quality thereof at high temperatures. Furthermore, when the content of the polyglycerol fatty acid ester mixture is less than 1 % by mass based on the total mass of ingredient (A), the effect of the polyglycerol fatty acid ester to make the dispersion state of lecithin uniform gets hard to obtain.

The total compounding amount of the polyglycerol fatty acid esters in the gel-form composition of the invention is preferably 0.01 to 10 % by mass, and more preferably 0.1 to 5 % by mass. When the compounding amount of the polyglycerol fatty acid esters is less than 0.01 % by mass, they cannot supplement the surface active properties of lecithin and it gets hard to disperse lecithin uniformly, and when it is more than 10 % by mass, the surface active properties of the polyglycerol fatty acid esters get too strong and high moisturizing properties characteristic of lecithin tend to be lost.

Polyglycerol fatty acid esters used in the invention may be generally available ones, but desirably are polyglycerol fatty acid esters prepared according to the process mentioned in JP-A-7-100355 or the process mentioned in JP-A-8-143513. The average polymerization degree of polyglycerols constituting polyglycerol fatty acid esters in the invention is that determined from the hydroxyl value in Methods for Analyzing and Testing Standard Oils and Fats (edited by Japan Society of Oil Chemistry). For measurement of the composition in polymerization degrees of polyglycerols constituting polyglycerol fatty acid esters, such a method is suitable that the composition of the polymerization degrees is determined by converting the polyglycerol fatty acid esters to their derivatives such as their trimethylsilyl ether (TMS) derivatives and then carrying out separation and quantitative determination by the GC method (gas chromatography). The measurement of HLB values can be made according to a generally published known method.

As lecithin used in the gel-form composition of the invention, there can be mentioned soy bean lecithin, rapeseed lecithin and yolk lecithin which are available as usual products on the market or reagents; and purified lecithin and fractionated lecithin obtained by subjecting such lecithin to process(es) such as solvent separation, extraction and/or fractionation; and further hydrogenated lecithin which is obtained by hydrogenating such lecithin to heighten saturation degree; and so on. Lecithin generally contains phosphatidylcholines having two straight-chain acyl groups each having 12 to 22 carbon atoms as main components, and phosphatidylethanolamines having two straight-chain acyl groups each having 12 to 22 carbon atoms and phosphatidylinositols having two straight-chain acyl groups each having 12 to 22 carbon atoms as minor components.
Particularly preferred among these lecithins is hydrogenated lecithin. The reason is that by hydrogenation, lecithin gets hard to deteriorate and thus the gel-form composition of the invention gets hard to deteriorate.

It is necessary that the compounding amount of lecithin in the gel-form composition of the invention is 1 % by mass or more. In less than 1 % by mass, the moisturizing properties which lecithin has cannot sufficiently be exerted, and gel-form compositions of such a low lecithin content are insufficient as moisturizing cosmetics and/or bases for them. There is no particular limitation about the upper limit of the compounding amount of lecithin, but it is preferred that the upper limit is on the order of 10.0 % by mass. When such lecithin that the concentration of phosphatidylcholine in the phospholipid is 20 % by mass or more, preferably 30 % by mass or more, and further preferably 50 % by mass or more is used, the effect to prevent secondary adhesion of the gel-form composition becomes more conspicuous.

As to physical properties of the gel-form composition of the invention, it is preferred that its loss elastic modulus G" value at 37 °C is 50 to 150 % of its G" value at 25 °C, and it is further preferred that its G" value at 37 °C is 70 to 130 % of its G" value at 25 °C. The loss elastic modulus G" value expresses viscosity, and when the above value is less than 50 % or more than 150 %, secondary adhesion after use of the gel-form composition is easy to cause, and a tendency for the qualities such as viscosity of the gel-form composition to change depending on the content of phosphatidylcholine in lecithin increases.

The loss elastic modulus G" value at 25 °C and the loss elastic modulus G" value at 37 °C correspond to the viscosity of the gel-form composition at ordinary temperature and the viscosity of the gel-form composition at the temperature at the time when it was applied to the human body, namely at body temperature, respectively. The difference in viscosity between these temperatures is considered to be caused by the change of the phase state at the time when the temperature rose in the course of application. It is considered that when the difference between the viscosities is large, the change of the phase state gets large and the gel-form composition goes into an unstable state, and thereby secondary adhesion after external use gets liable to occur. Particularly when the viscosity increases, it is considered that the gel-form composition becomes a more unstable system, and secondary adhesion after external use gets more liable to occur. This secondary adhesion after external use is considered not due to stickiness at external use but due to change of the state by change of the temperature. It is considered that when the difference in viscosity between these temperatures is small, phase change accompanying temperature rise is small and a stable state is maintained, and thus secondary adhesion after external use is prevented and it gets hard for the qualities such as viscosity to be influenced by the purity of phosphatidylcholine in lecithin.

In order to form a stronger gel state, it is possible to compound a saturated straight-chain aliphatic alcohol having 12 to 22 carbon atoms and/or a straight-chain monoalkyl ether of glycerol whose alkyl part has 12 to 22 carbon atoms to the gel-form composition of the invention, and there is no particular limitation about these ingredients so long as they are used in cosmetics and/or external preparations. As specific examples thereof, there can be mentioned lauryl alcohol, myristyl alcohol, cetanol, stearyl alcohol, cetostearyl alcohol, behenyl alcohol, monocetyl ether of glycerol (chimyl alcohol), monostearyl ether of glycerol (batyl alcohol), monobehenyl ether of glycerol, etc., and these can be used alone or in combination. In both of the saturated straight-chain aliphatic alcohol and the straight-chain monoalkyl ether of glycerol, when the number of the carbon atoms is less than 12, the effect of the gel-form composition to give hardness gets weak, and when it is more than 22, the texture of the gel-form composition gets very bad and value as a product is lowered.

In the case where such saturated straight-chain aliphatic alcohol and/or straight-chain monoalkyl ether of glycerol are/is used, it is suitable that the total compounding amount in the gel-form composition is 0.001 to 10 % by mass. In less than 0.001 % by mass, effect expected is not exerted, and in more than 10 % by mass, the gel-form composition gets too hard, and when applied, it comes to give touch of thick membrane, and further, moisturizing touch (humectant touch) brought about by lecithin tends to be lost.

It is desirable to compound a silicone oil to the gel-form composition of .the invention in an amount of 1 % by mass or more, preferably 1 % by mass or more but less than 5 % by mass of the composition. By compounding a silicone oil, the effect to inhibit secondary adhesion after external use is enhanced, but in less than 1 %, this effect is not sufficiently exerted. When it is compounded in an amount of 5% by mass or more, the sense of the silicone oil (remaining touch) remains on the coated part and secondary adhesion gets liable to occur.

As the silicone oil, there can be mentioned higher alkoxy-modified silicones, alkyl-modified silicones, higher fatty acid ester-modified silicones, etc. such as methylpolysiloxane, methylphenylpolysiloxane, methylhydrogenpolysiloxane, octamethylcyclopentasiloxane, dodecamethylcyclohexasiloxane and stearoxy-silicone, but the silicone oil is not limited thereto.

It is possible to incorporate other oily ingredients generally used in cosmetics and/or external preparations in the gel-form composition of the invention, and when they are used, it is preferred that the total compounding amount of said other oily ingredients in the gel-form composition of the invention is less than 5 % by mass. When they are compounded in an amount of 5 % by mass or more, the sense of said other oily ingredients (remaining touch) remains on the coated part and secondary adhesion gets liable to occur.

As said other oily ingredients, there can be mentioned natural animal and/or vegetable oils and fats, semisynthetic oils and fats, hydrocarbon oils, higher fatty acids, ester oils, glyceride oils, fat soluble vitamins, etc. Specific examples thereof include following ones, but are not limited thereto.

As natural animal and/or vegetable oils and fats, and semisynthetic oils and fats, there can be mentioned avocado oil, linseed oil, almond oil, olive oil, carnauba wax, candelilla wax, tallow, bovine leg fat, bovine bone fat, hardened tallow, wheat germ oil, sesame oil, rice germ oil, rice bran oil, safflower oil, soybean oil, camellia oil, evening primrose oil, corn oil, rapeseed oil, horse fat, palm oil, palm kernel oil, castor oil, hardened castor oil, sunflower seed oil, jojoba oil, macadamia nut oil, beeswax, mink oil, cotton seed oil, coconut oil, hardened coconut oil, peanut oil, lanolin, liquid lanolin, reduced lanolin, isopropyl lanolate, etc.

As hydrocarbon oils, there can be mentioned squalane, squalene, ceresin, paraffin, paraffin wax, liquid paraffin, microcrystalline wax, Vaseline, etc.

As higher fatty acids, there can be mentioned lauric acid, myristic acid, palmitic acid, stearic acid, behenic acid, undecylenic acid, oleic acid, linolic acid, linolenic acid, isostearic acid, 12-hydroxystearic acid, etc.

As ester oils, there can be mentioned diisobutyl adipate, 2-hexyldecyl adipate, di-2-heptylundecyl adipate, isostearyl isostearate, trimethylolpropane triisostearate, cetyl 2-ethylhexanoate, neopentyl glycol di-2-ethylhexanoate, trimethylolpropane tri-2-ethylhexanoate, pentaerythritol tetra-2-ethylhexanoate, cetyl octanoate, oleyl oleate, octyldodecyl oleate, decyl oleate, neopentyl glycol dicaprate, 2-ethylhexyl succinate, isocetyl stearate, butyl stearate, diisopropyl cebacate, cetyl lactate, myristyl lactate, 2-ethylhexyl palmitate, 2-hexyldecyl palmitate, 2-heptylundecyl palmitate, cholesteryl 12-hydroxystearate, phytosteryl oleate, diisostearyl malate, paramethoxycinnamic esters, pentaerythritol tetrarhodinate, etc.

As glyceride oils, there can be mentioned glycerol triisostearate, glycerol triisopalmitate, glycerol tri-(2-ethylhexanoate), glycerol trimyristate, glycerol diparamethoxycinnamate monoisooctanoate, etc.

As fat soluble vitamins, there can be mentioned tocopherols and their derivatives, retinol and its derivatives, etc.

Known ingredients used in the field of cosmetics can be compounded into the gel-form composition of the invention in such a range that the effects of the invention are not spoiled. For example, there can be mentioned aqueous ingredients such as disodium edetate; powder ingredients such as talc, silica, kaolin and magnesium oxide; moisturizing ingredients such as polyethylene glycol, propylene glycol, glycerol, 1,3-butylene glycol, 1,2-pentanediol, 1,2-hexanediol, 3-methyl-1,3-butanediol, xylitol, sorbitol, maltitol, chondroitin sulfuric acid, hyaluronic acid, mucoitin sulfuric acid, charonin sulfuric acid, atelocollagen, cholesteryl 12-hydroxystearate, sodium lactate, urea, salts of bile acid, salts of dl-pyrrolidonecarboxylic acid, short chain soluble collagen and diglycerol (EO) PO adducts; thickeners such as carboxyvinyl polymers, carboxymethylcellulose, polyvinyl alcohol and carrageenan; pH adjusting agents such as lactic acid-sodium lactate and citric acid-sodium citrate; antioxidants such as butylhydroxytoluene and sodium hydrogensulfite; antiseptics such as methylparaben and sodium benzoate; ultraviolet protectants such as paraaminobenzoic acid and octyl cinnamate; etc.

The gel-form composition of the invention can, for example, be prepared by mixing the above ingredients (A), (B) and (C), and, if necessary, oily ingredients, stirring the mixture to give a solution, and, if necessary, mixing other ingredients.

### EXAMPLES

The present invention is further explained in detail below according to examples. But, it goes without saying that the scope of the invention should not be limited by such examples. In the following mention, parts mean parts by mass unless otherwise defined.

### Examples 1 to 7 and Comparative examples 1 to 12

Gel-form compositions having compositions shown in Tables 1 to 3 were prepared. Each gel-form composition was prepared by mixing ingredients (1) to (6) and heating the mixture to around 80 °C, stirring the mixture for 10 minutes using a disper-mixer, adding ingredients (7) and (8) and then cooling the mixture.

On each gel-form composition, viscosity and secondary adhesion after external use were assessed. The viscosity was measured for seeing difference in quality due to the concentration of phosphatidylcholine in the lecithin, and the measurement was conducted according to The Japanese Standard of Cosmetic Ingredients (JSCI) method 2. Secondary adhesion after external use was assessed by firmly applying a certain amount (25 mg) of a gel-form composition onto the inside part (9 cm²) of the upper arm, pressing, one minute later, a glass sheet against the coated part, and visually observing the presence or absence of adhesion (4 steps × : there is a lot of adhesion, Δ: there is adhesion, ○: there is scarcely adhesion, ⓞ: there is no adhesion). In the invention, ⓞ and ○ are assumed to stand the test. The results are shown in Table 1 and Table 2.

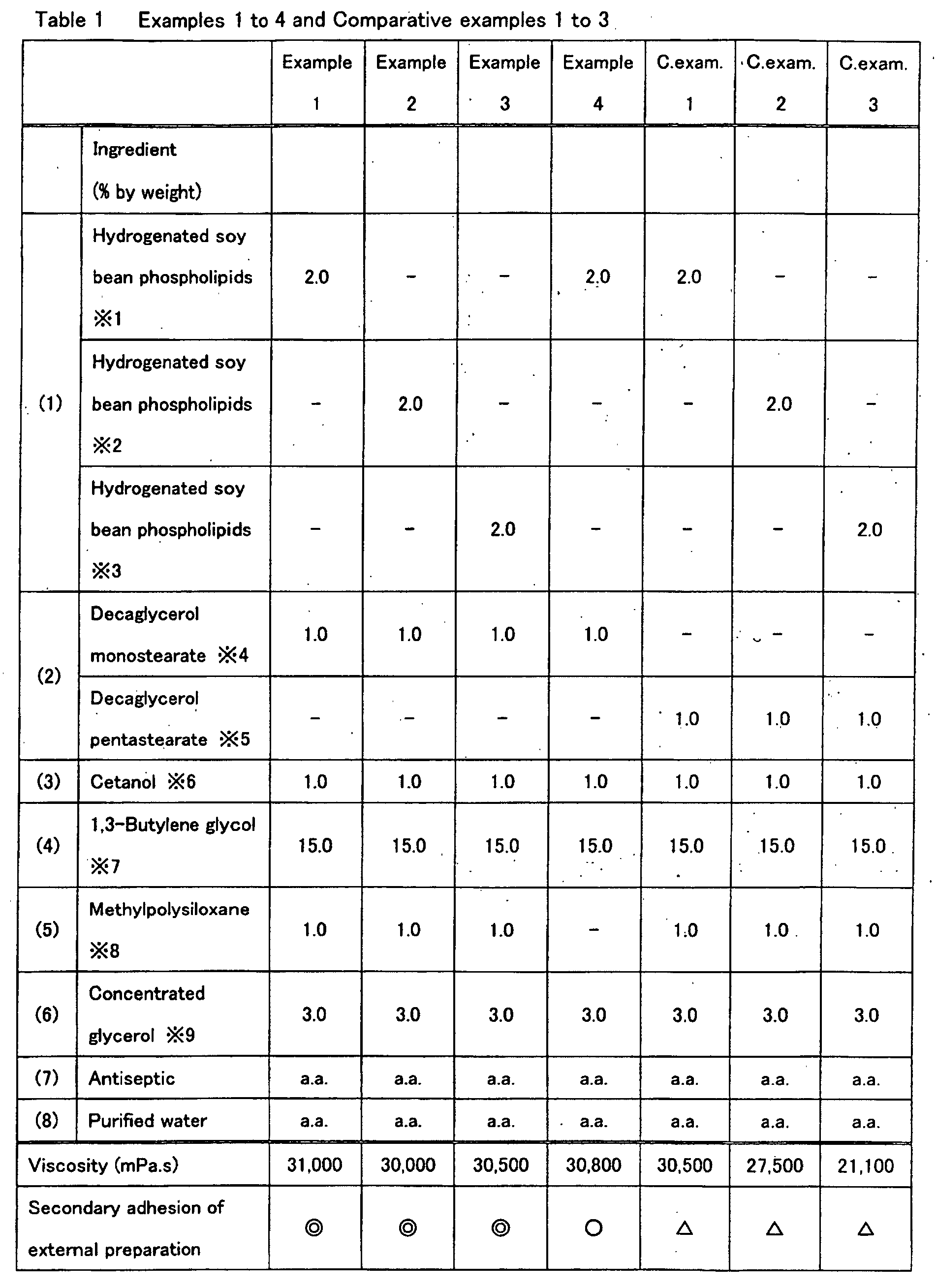

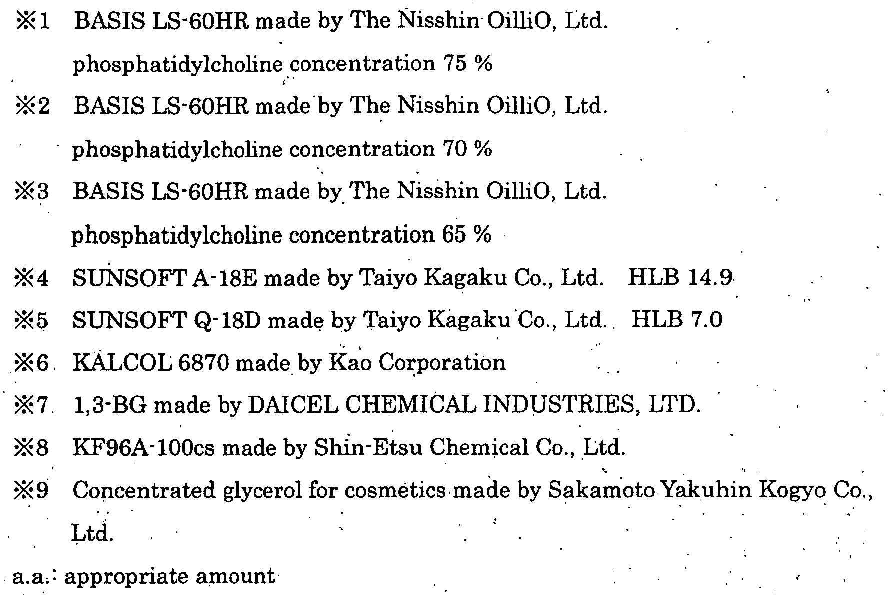

SUNSOFT A-18E is such a polyglycerol fatty acid ester mixture that the total content of polyglycerol fatty acid esters whose constitutive polyglycerols are pentamers of glycerol and polyglycerol fatty acid esters whose constitutive polyglycerols are hexamers of glycerol is 50 % by mass or more and the total content of polyglycerol fatty acid esters whose constitutive polyglycerols are tetramers and less oligomers of glycerol is less than 20 % by mass.

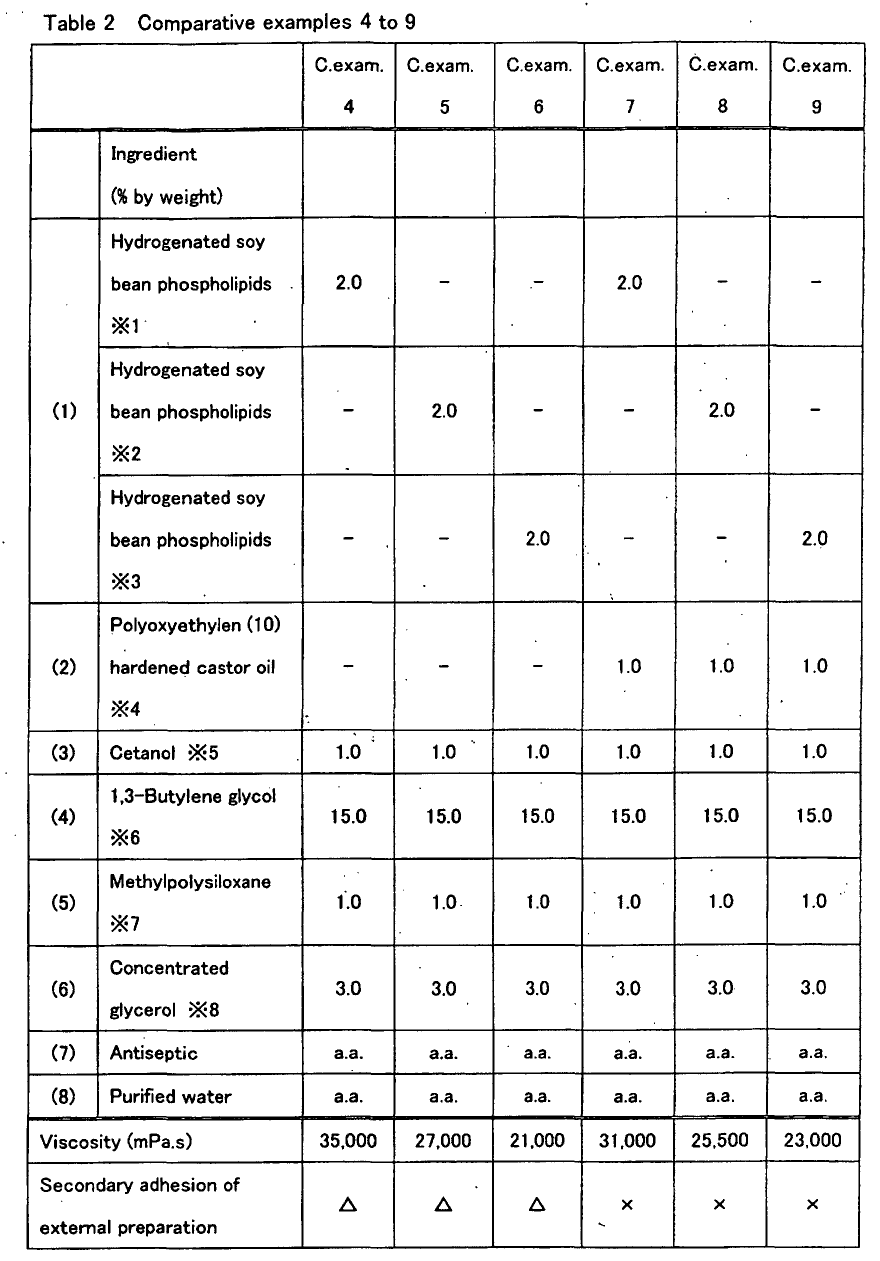

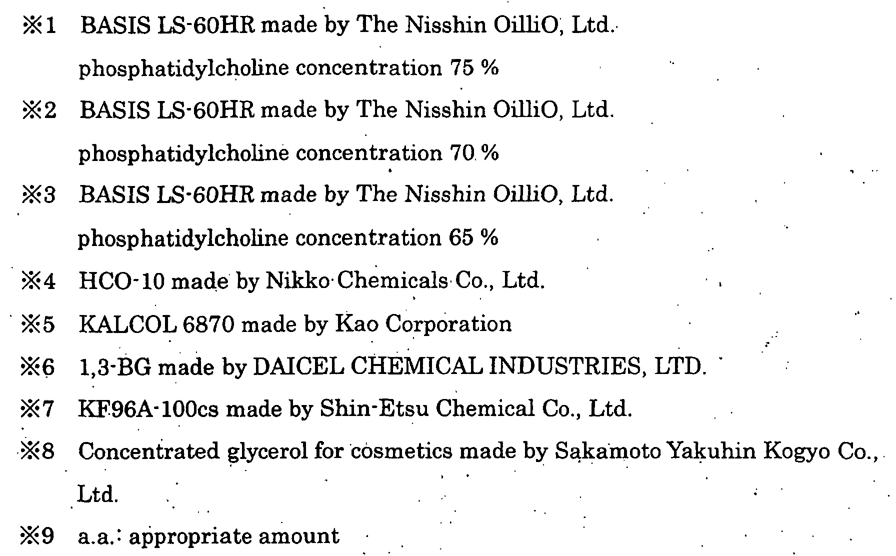

As apparent from Table 1 and Table 2, it is seen that, in gel-form compositions using lecithin as a base, the products of the present invention (Examples 1 to 4) prevent secondary adhesion after external use and retain stable quality (=viscosity) regardless of the purity of phosphatidylcholine in the lecithin, in comparison with those of the prescription system where no surfactant was compounded (Comparative examples 4 to 6), those of the prescription system where the ethylene oxide-type surfactants were compounded (Comparative examples 7 to 9) and those of the prescription system where the polyglycerol fatty acid esters other than ones specified in the present invention were compounded (Comparative examples 1 to 3). Further, when the ethylene oxide-type surfactants were compounded, secondary adhesion after external use was conspicuous compared with the system where no surfactant was compounded (comparison between Comparative examples 4 to 6 and Comparative examples 7 to 9). Further, it is seen from comparison between Example 1 and Example 4 that the silicone oil enhanced the effect to prevent secondary adhesion after external use.

Gel-form compositions of lecithin in which no surfactant was compounded, ethylene oxide-type surfactants were compounded, or polyglycerol fatty acid esters were compounded were checked for viscosity (loss elastic modulus G"), and the results are shown below. The loss elastic modulus G" of each gel-form composition was measured using Rheo Stress RS-1, a viscosity and viscoelasticity measuring apparatus, made by HAAKE Corporation. Temperature was raised at a rate of 2 °C per minute from 5 to 60 °C under a certain stress (1 Pa) and a certain frequency (1 Hz), and G" values at 25 °C and 37 °C were measured. Their change amounts {(G" value at 37 °C / G" value at 25 °C) × 100 (%)} were calculated. The results are shown in Table 3 together with the test results of secondary adhesion after external use.

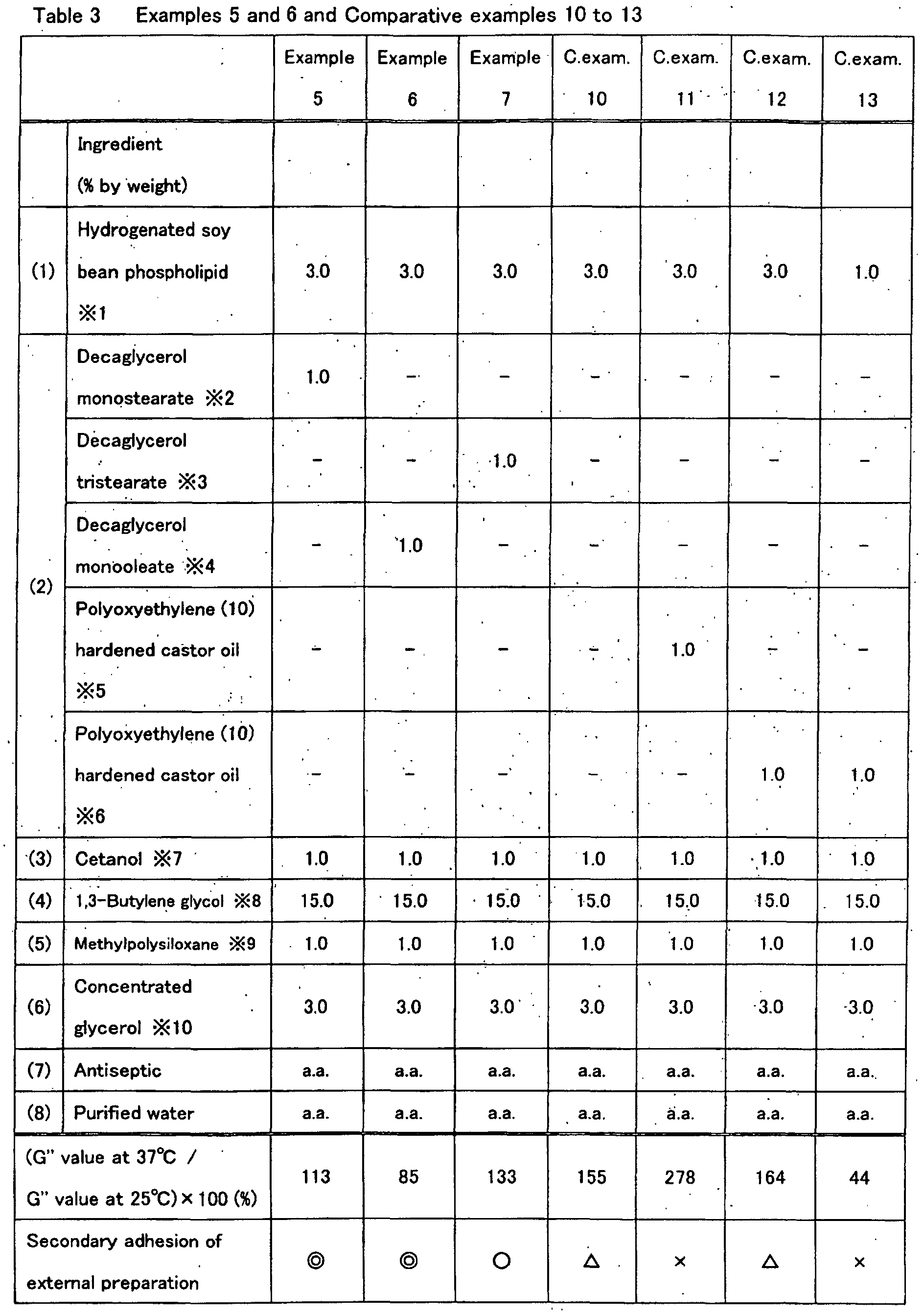

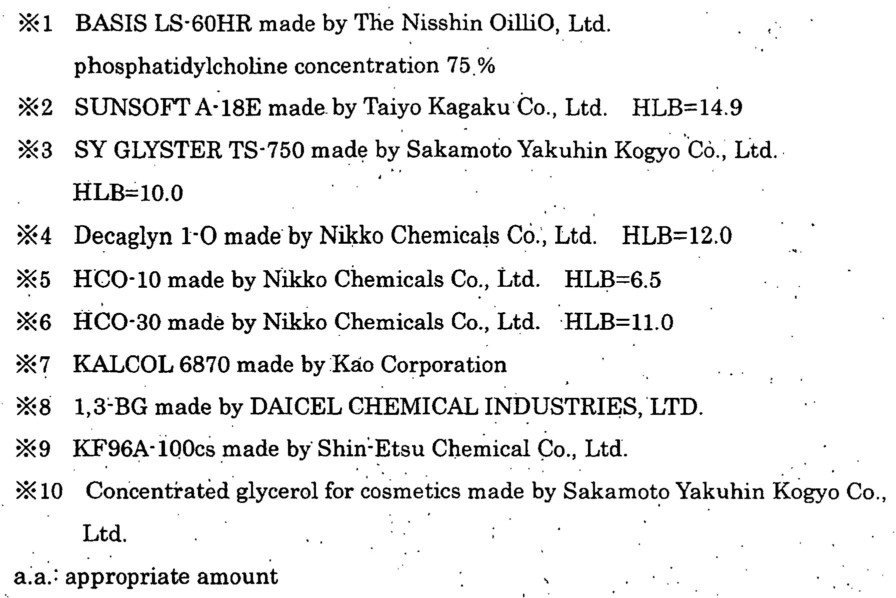

As apparent from Table 3, it is seen that in the products of the present invention (Examples 5 to7) in comparison with that of the prescription system where no surfactant was compounded (Comparative example 10) and those of the prescription system where the ethylene oxide-type surfactants were compounded (Comparative examples 11 to 13), the difference between the values of loss elastic modulus at 25 °C and 37 °C is small. This means that when temperature is raised from ordinary temperature to body temperature, the change of the phase state is small, and thus it is considered that the products of the present invention are stable systems and as a result secondary adhesion after external use scarcely occurs. It is considered that the reason why the difference between the values of loss elastic modulus (viscosity) at 25 °C and 37 °C is large in the prescription system where the ethylene oxide-type surfactants were compounded and the prescription system where no surfactant was compounded is that, in the compositions in which the ethylene oxide-type surfactants were compounded, their viscosities are liable to be influenced by the change of temperature, and, in the composition in which no surfactant was compounded, since the composition is an unstable system where lecithin is not uniformly dispersed, the state of the phase formed from water and lecithin gets easy to destroy due to rise of temperature, compared with the systems of uniform dispersion, and as a result, the viscosities of these gel-form compositions change largely.

As to improvement (reduction) of touch of thick membrane and stickiness, which is the effect aimed at by gel-form compositions, aforementioned as prior art, which use lecithin as a base and wherein ethylene oxide-type surfactants are compounded, the gel-form compositions of the present invention (Examples 1 to 7) were superior to the gel-form compositions where no surfactant was compounded (Comparative examples 4 to 6 and 10), and were equal to the gel-form compositions where other polyglycerol fatty acid esters than those specified in the present invention were compounded (Comparative examples 1 to 3) and the gel-form compositions where ethylene oxide-type surfactants were compounded (Comparative examples 7 to 9 and 11 to 13).

### INDUSTRIAL APPLICABILITY

The gel-form composition of the invention can prevent external preparations or the like after external use from adhering to other things when the coated parts contact with said other things, so-called from secondary adhesion, and, at the same time, can retain stable quality regardless of the purity of phosphatidylcholine in the lecithin. The gel-form composition of the invention also has an effect to improve touch of thick membrane and stickiness at the time of application. Therefore, by using the gel-form composition of the invention, it was made possible to provide gel-form compositions which have a wide use range and can be adjusted to the same quality. The gel-form composition of the invention can, for example, be used as moisturizing cosmetics as applied to hands, faces, etc. or as their base.

## Claims

1. A gel-form composition comprising the following ingredients (A), (B) and (C) as indispensable ingredients, the compounding amount of lecithin in the gel-form composition being 1 % by mass or more:
(A) a polyglycerol fatty acid ester having an HLB value of 8 or more, each fatty acid residue thereof having 14 to 22 carbon atoms,
(B) lecithin, and
(C) water.

2. The gel-form composition according to claim 1 wherein the average polymerization degree of glycerol in the polyglycerol constituting ingredient (A) is 3 to 12.

3. The gel-form composition according to claim 1 or 2 wherein the compounding amount of ingredient (A) in the gel-form composition is 0.01 to 10 % by mass.

4. The gel-form composition according to any one of claims 1 to 3 wherein, as to the value of the loss elastic modulus G" of the gel-form composition, the value of the loss elastic modulus G" at 37°C is 50 to 150 % of the value at 25°C.

5. The gel-form composition according to claim 4 wherein the value of the loss elastic modulus G" at 37°C is 70 to 130 % of the value at 25°C.

6. The gel-form composition according to any one of claims 1 to 5 wherein ingredient (A) contains such a polyglycerol fatty acid ester mixture that the total content of polyglycerol fatty acid esters whose constitutive polyglycerols are pentamers of glycerol and polyglycerol fatty acid esters whose constitutive polyglycerols are hexamers of glycerol is 50 % by mass or more and the total content of polyglycerol fatty acid esters whose constitutive polyglycerols are tetramers and less oligomers of glycerol is less than 20 % by mass; the content of the polyglycerol fatty acid ester mixture being 1% by mass or more based on the total mass of ingredient (A).

7. The gel-form composition according to any one of claims 1 to 6 wherein the lecithin is hydrogenated lecithin.

8. The gel-form composition according to any one of claims 1 to 7 wherein the lecithin is such that its phosphatidylcholine content is 20 % by mass or more.

9. The gel-form composition according to any one of claims 1 to 8 wherein a silicone oil is compounded so that the silicone oil content in the gel-form composition may be 1 % by mass or more.
